# EUROPEAN PATENT SPECIFICATION

(11) **EP 0 908 467 B1**
(45) Date of publication and mention of the grant of the patent: **22.01.2003**
(21) Application number: 97928452.8
(22) Date of filing: 24.06.1997
(51) Int. Cl.: C08B 37/00, A61K 31/715, A61K 31/737

(54) **USE OF THE HOT-WATER EXTRACT OF ''NEMACYSTUS DECIPIENS''**
VERWENDUNG DES HEISSWASSER-EXTRAKTS VON ''NEMACYSTUS DECIPIENS''
UTILISATION D'UN EXTRAIT DE ''NEMACYSTUS DECIPIENS'' OBTENU AU MOYEN D'EAU CHAUDE

(30) Priority: 01.07.1996 JP 17102096
(43) Date of publication of application: 14.04.1999
(73) Proprietor: OTSUKA PHARMACEUTICAL FACTORY, INC., Naruto-shi, Tokushima 772-8601 (JP)
(72) Inventor: SATO, Takaya, Tokyo 123 (JP); UEHARA, Tsutomu, Saitama 343 (JP); YAMAOKA, Ippei, Tokushima 772 (JP); ASAGI, Kozo, Tokushima 772 (JP); KOHRI, Hideaki, Kitajima-cho, Itano-gun Tokushima 771-02 (JP)
(74) Representative: HOFFMANN - EITLE
(86) International application number: JP9702182
(87) International publication number: WO98000447

(56) References cited:
- EP-A- 0 295 956
- EP-A- 0 645 143
- JP-A- 49 057 000
- JP-A- 57 202 302
- CHEMICAL ABSTRACTS, vol. 97, no. 10, 6 September 1982 (1982-09-06) Columbus, Ohio, US; abstract no. 75395, T. FUJIKAWA AND M. WADA: "FUCOIDAN AND FUCOIDAN-LIKE SUBSTANCES" XP002125517 & AGRIC. BIOL. CHEM., vol. 39, no. 5, 1975, page 1109-1114
- CHEMICAL ABSTRACTS, vol. 106, no. 13, 30 March 1987 (1987-03-30) Columbus, Ohio, US; abstract no. 99044, B. KLOAREG ET AL.: "POLYANIONIC CHARCTERISTICS OF PURIFIED ... FROM BROWN ALGAE" XP002125518 & INT. J. BIOL. MACROMOL., vol. 8, no. 6, 1986, page 380-386
- PATENT ABSTRACTS OF JAPAN vol. 007, no. 268 (C-197), 30 November 1983 (1983-11-30) & JP 58 149666 A (NAGOYA SEIRAKU KK), 6 September 1983 (1983-09-06)
- PATENT ABSTRACTS OF JAPAN vol. 011, no. 297 (C-448), 25 September 1987 (1987-09-25) & JP 62 087071 A (NAGAOKA KORYO KK;OTHERS: 01), 21 April 1987 (1987-04-21)

## Description

### TECHNICAL FIELD

The present invention relates to the use of a hot-water extract of a seaweed of the family *Spermatochnaceae.* More specifically, the present invention relates to the use of a hot-water extract of a seaweed of the family *Spermatochnaceae* excellent in the action of adsorbing excessively-ingested sodium ions in the gastro-intestinal tracts to promote the excretion thereof into feces; and use thereof for foods and pharmaceuticals.

### BACKGROUND OF THE INVENTION

EP 0 645 143 A discloses an anti-ulcer agent comprising fucoidan as an active ingredient. This agent may be obtained by extracting red algae such as *Phaeophyceae Chordariales nemacystus* and brown algae such as *Padina arborescens, Undaria pinnatifida, Fucus evanescens, Himanthulia, Bifurcaria and Fucus vesiculosus.*

EP 0 295 956 A discloses polysaccarides which are obtainable by extracting marine algae with an aqueous solvent. The polysaccaride can be used as an antiviral agent. A preferred embodiment is directed to the use in the treatment of AIDS.

According to the National Nutrition Survey Results published by the Ministry of Health and Welfare, Japanese have taken at least 11.5 g of salt a day since 1975, and particularly in 1993, a daily *salt* intake was 12.8 g. Since there is a correlation between a daily salt intake and a crisis rate of hypertension, the Ministry of Health and Welfare is recommending to decrease the daily salt intake to 10 g or less so as to prevent the crisis of hypertension and moreover, stroke and the like. In the U.S., a daily salt intake has been limited similarly and the recommendation of the U.S. Joint Committee proposes that a daily salt intake of patients suffering from hypertension must be controlled to 6 g or less.

It is said that there is also a correlation between a salt intake and a mortality from gastric cancer. According to the data obtained so far, in a district where a salt intake is large, for example, Toyama city or Hirosaki city, the mortality from gastric cancer is high; on the other hand, in a district where a salt intake is small, for example, Beppu city or Okinawa city, the mortality from gastric cancer is low.

It is reported that dietary fibers such as alginate have sodium adsorbing capability to some extent (*Journal of Home Economics of Japan,* vol. 39, No. 3, p. 187-195 (1988)), but its adsorbing capability was still insufficient.

The excessive salt intake thus has a bad influence on the human body. Accordingly, a strong demand for the development of new technique to extracorporeally excrete the excessively ingested salt is required.

Accordingly, an object of the present invention is to provide a component which extracorporeally excrete the excessively ingested salt positively and safely, *and* to provide foods and pharmaceuticals containing the component.

### DISCLOSURE OF THE INVENTION

The present inventors have carried out various investigations to find a component having the above-described action from a wide range of food materials. As a result, it has been found that a hot-water extract of a seaweed of the family *Spermatochnaceae* has excellent sodium ion adsorbing capability and excretion promoting capability, and that it can be made use of as foods or pharmaceuticals. Thus, the present invention has been completed.

That is, the present invention teaches the use of a hot-water extract of a seaweed of the family *Spermatochnaceae (Mozuku)* having a weight average molecular weight of at least 30,000 for the preparation of an agent for preventing or treating a disease induced by excessive salt intake such as hypertension, gastric cancer or stroke.

Preferred embodiments of the present invention are specified in the dependent claims.

### BEST MODE FOR CARRYING OUT THE INVENTION

The seaweed of the family *Spermatochnaceae* for use in the present invention is a kind of seaweeds and is edible as food. The extract of the seaweed of the family *Spermatochnaceae is* known to have serum lipid lowering action (*Nagahara Gakuen Nishi-kyushu University, Saga Junior College Bulletin,* No. 25, p. 171-173 (1995)), but hot-water extraction of an effective ingredient from the seaweed of the family *Spermatochnaceae* and action of the resulting extract on sodium ions are not known.

Examples of the seaweed of the family *Spermatochnaceae* for use in the present invention include *Tinocladia crassa, Nemacystus decipiens* and *Cladosiphon okamuranus. An* extract from the seaweed of the family *Spermatochnaceae* can be obtained, for example, by extracting it with hot water of 60 to 100°C, preferably 80 to 90°C, for 30 minutes to 3 hours, preferably for about 1 hour, and then removing the low-molecular weight substances by dialysis. The resulting hot-water extract may be used as is or if necessary, after purified through alcohol precipitation, ion exchange resin chromatography, or gel filtration chromatography.

The preferable physical properties of the resulting hot-water extract are as follows:
(1) Weight-average molecular weight: 50,000 to 500,000
(2) Neutral sugar content: 50 to 90%
(3) Sulfur content: 3 to 17%

Since the main component of the hot-water extract is a sugar chain, it is considered that fucoidan is contained therein. However, as shown in the following Test Example, the hot-water extract of the present invention has more markedly excellent sodium ion adsorbing capability than fucoidan purified even to about 100% purity. Therefore, a substance other than fucoidan would be contained in the hot-water extract of the present invention, and this substance would have enhancing action on sodium ion adsorbing capability.

The hot-water extract to be used according to the present invention has sodium ion adsorbing capability. Particularly, when orally administered, it has action of adsorbing sodium ions in the gastro-intestinal tracts to promote its excretion. Accordingly, it is useful as a pharmaceutical or food for preventing or treating various diseases induced by an excessive salt intake, for example, hypertension, gastric cancer and stroke.

The food or pharmaceutical containing the hot-water extract of the present invention can be prepared by processing the hot-water extract of a seaweed of the family *Spermatochnaceae*, which has been obtained by the above-described process, into various forms by the conventional manner. Examples of the forms include solid, liquid, emulsified and paste forms.

Examples of the form as a pharmaceutical include tablets, powders, granules, fine particles and liquids. These preparations can be prepared by treating the hot-water extract of the present invention together with a pharmaceutically acceptable carrier in a conventional manner.

Examples of the food according to the present invention include an edible food without any treatment, an edible food after cooking or the like, and a premixed material for food production. The solid food may be any of solid, powder and granular forms, such as various confectioneries (e.g., biscuits, cookies, cakes, crisps and rice crackers), bread, and powdered drinks (e.g., coffee beverage powder and cocoa beverage powder). Examples of the food in the liquid, emulsified or paste form include various beverages, such as juice, carbonated beverages, and lactobacillus beverages. Among these, the beverages are particularly preferred in the present invention.

About 1 g of the hot-water extract of a seaweed of the family *Spermatochnaceae* according to the present invention has capability of adsorbing about 150 mg of salt. As a result of calculation in terms of the seaweed of the family *Spermatochnaceae* based on the above value, it is preferred to take about 10 to 20 g of the hot-water extract of the seaweed of the family *Spermatochnaceae* a day.

### EXAMPLES

In order to demonstrate the present invention more specifically, the process for obtaining the hot-water extract from the seaweed of the family *Spermatochnaceae* according to the present invention and processes for producing a pharmaceutical and food containing this hot-water extract will be described in Examples, and Test Examples using the hot-water extract of the seaweed of the family *Spermatochnaceae* obtained in these examples will also be described. However, the present invention is not limited thereto.

### Example 1

Edible *Nemacystus decipiens* from Ise-Shima was used as a raw seaweed. The raw seaweed *Nemacystus decipiens* was washed with water to remove impurities attached to the surface of *Nemacystus decipiens*. Water was removed through a sieve, and 252 g of the wet *Nemacystus decipiens* was weighed and provided for extraction. The water content of the wet *Nemacystus decipiens* was measured by an infrared heating type moisture meter, resulting in 95.4%. Together with 1200 ml of water, 252 g of *Nemacystus decipiens* was pulverized in a Waring Blender. The resulting solution was maintained at 90°C for 1 hour for hot-water extraction treatment. The resulting solution was allowed to cool down to room temperature and centrifuged (15000 G, 10 min) to remove the water insoluble matters. The supernatant was transferred to a cellulose-made dialysis tube and dialyzed against ion exchange water to remove low-molecular weight substances having a molecular weight of 10,000 or less. The dialyzed solution was centrifuged again (15000G, 10 min) to remove the insoluble matters completely. By lyophilization of the supernatant, 2 g of the hot-water extract of *Nemacystus decipiens* was obtained.

### (1) Measurement of molecular weight

A liquid chromatographic pump ("CCCP", produced by TOSOH CORPORATION), gel filtration column ("TSK gel GM-PWXL", produced by TOSOH CORPORATION), column oven ("CO-8020", produced by TOSOH CORPORATION), differential refractometer ("RI-410", produced by Waters Co., Ltd.) and degasser ("SD-8022", produced by TOSOH CORPORATION) were used. With a 0.1 M aqueous solution of sodium chloride as a mobile phase, the molecular weight was measured at a temperature of 40°C and a flow rate of 0.8 ml/min. The retention time was compared with that of the standard Pullulan sample (produced by Showa Denko K.K.) and the weight-average molecular weight was calculated using a chromatographic integrator ("Chromatocoder 21", produced by TOSOH CORPORATION).

The hot-water extract of *Nemacystus decipiens* as measured in the above-described manner had a weight-average molecular weight of 50,000 to 500,000.

### (2) Measurement of neutral sugar content

As reagents, (a) concentrated sulfuric acid (reagent grade: the highest quality) and (b) a 5% aqueous solution of phenol (fresh one which does not assume yellowish color) were employed. Fucose (reagent grade: the highest quality) was dissolved in pure water to obtain solutions having precisely a concentration of 20 µg to 600 µg/ml. From these, five solutions different in the concentration were selected. In a *test* tube, 0.5 ml of each of the five solutions was charged, and 0.5 ml of the above-described reagent (b) was added. The resulting mixture was stirred sufficiently. Then, to the reaction mixture, 2.5 ml of the above-described reagent (a) was added, followed by stirring. After the reaction mixture was allowed to stand at room temperature for 15 minutes, the absorbance at 480 nm was measured by an absorption spectrophotometer. The calibration curve of the neutral sugar concentration and absorbance at 480 nm was prepared. Then, 150 µg of the above-described extract *of Nemacystus decipiens* was weighed accurately and dissolved in 10 ml of pure water. In a test tube, 0.5 ml of the resulting solution was poured, and 0.5 ml of the reagent (b) was added. The resulting mixture was stirred sufficiently. To the reaction mixture, 2.5 ml of the reagent (a) was added, and the resulting mixture was stirred. After the reaction mixture was allowed to stand at room temperature for 15 minutes, the absorbance at 480 nm was measured by an absorption spectrophotometer. From the calibration curve prepared in advance, the concentration of the neutral sugar was calculated. Concerning the sample to be used for the measurement of the neutral sugar content, the water content thereof was calculated by an infrared moisture meter, and the neutral sugar content was analyzed after gravimetric correction.

The neutral sugar content of the hot-water extract of *Nemacystus decipiens* as measured in the above-described manner was 50 to 90%.

### (3) Measurement of sulfur content

The sulfur content of the hot-water extract of *Nemacystus decipiens* was analyzed by burning, thereby oxidizing the sample in oxygen and determining the resulting sulfur oxide by precipitation titration. This method was carried out in accordance with JIS K-0103. The sulfur content is defined as (sulfur weight/sample weight) × 100.

The hot-water extract of *Nemacystus decipiens* as measured by the above-described method had a sulfur content of 3 to 17%.

### Example 2

### Preparation of fine particles:

A mixture of 70 parts by weight of the hot-water extract of *Nemacystus decipiens* of the present invention, which had been prepared in Example 1, 20 parts by weight of lactose and 10 parts by weight of corn starch were subjected to fluidized bed granulation with a 5% aqueous solution of hydroxypropylmethyl cellulose to obtain fine particles.

### Example 3

### Preparation of biscuits:

In the following examples, "parts" are by weight.

To a mixture of 8 parts of shortening and 18 parts of sugar, 42 parts of soft flour, 7.5 parts of the hot-water extract of *Nemacystus decipiens of* the present invention which had been obtained in Example 1, 0.8 part of baking powder, 16 parts of an egg, 1 part of glucose and 25 parts of water were added, followed by stirring to obtain a dough. The dough was pressed to have a thickness of 5 mm and cut out into pieces, each 16 to 17 g in weight. The piece was baked in an oven of 90°C for 32 to 36 minutes to obtain a biscuit of about 12 g. According to the calculation, the biscuit of 12 g contains 1 g hot-water of the *Nemacystus decipiens* extract. That is, this biscuit can adsorb thereto about 60 mg of sodium ions (about 150 mg as a salt), when ingested.

### Example 4

### Preparation of beverage:

A bottle was filled with 12.5 g of granulated sugar, 0.2 g of citric acid crystals, 1 g of the hot-water extract of *Nemacystus decipiens* of the present invention obtained in Example 1 and the balance of ion exchange water to give 100 ml in total, and sterilized at 80°C for 10 minutes to obtain a *Nemacystus-decipiens*-extract-containing beverage.

### Test Example 1

### (1) Preparation of salt solution

Na, K and Ca ion concentrations in the small intestine fluid are 119, 4.2 and 4 (mEq/L), respectively. Relating to a daily mean ion intake of the Japanese, Na, K and Ca are 5072, 2700 and 541 mg, respectively. The intake per meal is obtained by dividing them by three, resulting in 1691, 900 and 180.3 mg, respectively.

If the water contents in the stomach and duodenum after meal are 0.75 L and 0.5 L, respectively, the salt concentrations of Na, K and Ca are 58.8, 18.5 and 7.2, respectively. When the above-described salt concentrations in the small intestine fluid are added, the final salt solution concentration of Na, K and Ca in the small intestine become 89, 11.4 and 5.6 mM, respectively.

### (2) Preparation of a Ca salt of Fucoidan

In a 1-L beaker containing 500 ml of D. W., 1.0 g of fucoidan (produced by Sigma Chemical Co.) was charged, stirred with a stirrer, and completely dissolved. In the resulting solution, 10.0 g of calcium chloride dihydrate (Katayama Chemicals, Inc.) was charged and the resulting mixture was stirred, and completely dissolved. The reaction mixture was allowed to stand for 2 hours to complete the calcium-salt-forming reaction of fucoidan. From the resulting sugar solution, the resulting salt was removed through a cellulose dialysis membrane (3.5 mm diameter, produced by Sanko Junyaku Co., Ltd.), and lyophilized to obtain 1.042 g of the Ca salt of fucoidan.

### (3) Measurement of degree of Na ion bond

Several drops of concentrated hydrochloric acid were added to 50 ml of the salt solution prepared in the above (1) to adjust the pH of the solution to 1.2. To the resulting solution, 1 g of the hot-water extract of *Nemacystus decipiens* of the present invention obtained in Example 1, and 1 g of the Ca salt of fucoidan and 1 g of the calcium alginate ("Flavikafine", trade name; produced by Nisshinbo Industries, Inc.), which were control substances, were dissolved. Under stirring, the resulting solution *was* allowed to stand at room temperature for 60 minutes. To the reaction mixture, several drops of a concentrated aqueous solution of sodium hydroxide were added to adjust its pH to 8.0. Under stirring, the reaction mixture was allowed to stand at room temperature for 60 minutes. Then, the each ion exchange resin was removed by filtration through an ultrafiltration membrane having a cutoff molecular weight of 5,000. The ion concentration in the filtrate was determined by atomic absorption analysis.

### (4) Results

The Ca salt of fucoidan, which was a control substance, adsorbed 16.37 (mg/resin g) of sodium ions. The calcium alginate, which was a control substance, adsorbed 34.5 (mg/resin g) of sodium ions.

On the other hand, the hot-water extract of *Nemacystus decipiens* according to the present invention adsorbed 49.87 (mg/resin g) of sodium ions and exhibited higher adsorbing capability than the control substances.

### Test Example 2

As experimental animals, 5-week-old Wistar male rats (Charles River Japan) were employed. After preliminary feeding with a commercially available solid feed ("CRF1", Oriental Yeast Co., Ltd.) for 5 days, the rats were transferred to a metabolic cage. They were subjected to preliminary feeding with a powdery feed CRF1. Then, they were weighed and classified into groups (n=5) using the weight so measured and feed intake during preliminary feeding as indexes. The crude extract of *Nemacystus decipiens* was employed as a test drug and they were fed for 7 days with the test-drug-containing feed having the composition as shown below in Table 1. Similarly, cellulose ("Cellulose powder", trade name, produced by Oriental Yeast Co., Ltd.) as a negative control and the calcium alginate ("Flavikafine", trade name; produced by Nisshinbo Industries, Inc.) as a positive control were used for the experiment. During the test, the feed intake and water intake were measured every day. From the third day after the feeding with the test-drug-containing feed, feces were collected every 24 hours for 4 days and provided for use in the measurement of Na.

After variance analysis, the values of each group so measured were detected by the Tukey-Kramer method and the significant difference was set at p < 0.01.

**Table 1**

| Composition of feed (%) | |
|---|---|
| Casein | 20 |
| α-Cornstarch | 49.5 |
| NaCl | 1 |
| Sucrose | 10 |
| Cellulose powder | 5 |
| Vitamin mix ∗1 | 1 |
| Mineral mix ∗2 | 3.5 |
| Corn oil | 5 |
| Test sugar | 5 |

| | |
|---|---|
| ∗1: AIN-76 vitamin mixture (containing choline bitartrate) | |
| ∗2: AIN-76 mineral mixture | |

**Table 2**

| Mineral content of each test drug (mg/100 g) | | | |
|---|---|---|---|
| Test drug | Na | K | Ca |
| Cellulose | 513 | 352 | 513 |
| Ca salt of alginic acid | 514 | 352 | 903 |
| Hot-water extract of *Nemacystus decipieus* of the present invention in Example 1 | 544 | 359 | 733 |

### Results:

The ratio of the amount of sodium excreted in the feces to the sodium intake (hereinafter referred to as "excretion ratio") was measured. As a result, the sodium excretion ratio of the *Nemacystus decipiens* crude extract group of the present invention (10.1%) was significantly higher than the group of cellulose (0.4%) or calcium alginate (1.4%).

### INDUSTRIAL APPLICABILITY

The hot-water extract of a seaweed of the family *Spermatochnaceae* adsorbs sodium ions in the gastro-intestinal tracts and promotes their excretion so that foods or pharmaceuticals containing it are useful for the preparation of an agent for the prevention or treatment of diseases induced by an excessive salt intake.

## Claims

1. The use of a hot-water extract of a seaweed of the family *Spermatochnaceae (Mozuku)* having a weight average molecular weight of at least 30,000 for the preparation of an agent for preventing or treating a disease induced by excessive salt intake.

2. The use according to claim 1, wherein the disease is hypertension, gastric cancer or stroke.

3. The use according to claim 1 or 2, wherein the hot-water extract has a weight-average molecular weight of 50,000 to 500,000 and a neutral sugar content of 50 to 90%.

4. The use according to claim 3, wherein the hot-water extract has a sulfur content of 3 to 17%.

5. The use according to any one of claims 1 to 4, wherein the agent is a pharmaceutical or food.

6. The use according to any one of claims 1 to 5, wherein the agent is in the form of a solid, a liquid, an emulsion or a paste.

7. The use according to any one of claims 1 to 6, wherein the agent is a beverage.

## Patentansprüche

1. Verwendung eines Heißwasser-Extrakts aus Seetang der Familie der Spermatochnaceae (Mozuku) mit einem gewichtsdurchschnittlichen Molekulargewicht von mindestens 30000 zur Herstellung eines Mittels zur Vorbeugung und Verhinderung oder Behandlung von Krankheiten, die durch übermäßige Salz-Einnahme induziert werden.

2. Verwendung gemäß Anspruch 1, wobei die Krankheit BlutHochdruck, Magenkrebs oder Schlaganfall ist.

3. Verwendung gemäß Anspruch 1 oder 2, worin der Heißwasser-Extrakt ein gewichtsdurchschnittliches Molekulargewicht von 50000 bis 500000 und einen Neutralzucker-Gehalt von 50 bis 90% aufweist.

4. Verwendung gemäß Anspruch 3, worin der Heißwasser-Extrakt einen Schwefel-Gehalt von 3 bis 17% aufweist.

5. Verwendung gemäß einem der Ansprüche 1 bis 4, wobei das Mittel ein Pharmazeutikum oder Nahrungsmittel ist.

6. Verwendung gemäß einem der Ansprüche 1 bis 5, wobei das Mittel in Form eines Feststoffs, einer Flüssigkeit, einer Emulsion oder einer Paste vorliegt.

7. Verwendung gemäß einem der Ansprüche 1 bis 6, wobei das Mittel ein Getränk ist.

## Revendications

1. Utilisation d'un extrait à l'eau chaude d'une algue de la famille des Spermatochnacées *(Mozuku)* ayant une masse moléculaire moyenne en poids d'au moins 30 000 pour la préparation d'un agent destiné à prévenir ou traiter une maladie induite par une absorption excessive de sel.

2. Utilisation selon la revendication 1, dans laquelle la maladie est l'hypertension, le cancer de l'estomac ou une attaque.

3. Utilisation selon la revendication 1 ou la revendication 2, dans laquelle l'extrait à l'eau chaude a une masse moléculaire moyenne en poids de 50 000 à 500 000 et une teneur en sucres neutres de 50 à 90%.

4. Utilisation selon la revendication 3, dans laquelle l'extrait à l'eau chaude a une teneur en soufre de 3 à 17%.

5. Utilisation selon l'une quelconque des revendications 1 à 4, dans laquelle l'agent est un produit pharmaceutique ou alimentaire.

6. Utilisation selon l'une quelconque des revendications 1 à 5, dans laquelle l'agent se présente sous la forme d'un solide, d'un liquide, d'une émulsion ou d'une pâte.

7. Utilisation selon l'une quelconque des revendications 1 à 6, dans laquelle l'agent est une boisson.
